# EUROPEAN PATENT SPECIFICATION

(11) **EP 0 438 704 B1**
(45) Date of publication and mention of the grant of the patent: **08.03.1995**
(21) Application number: 90123998.8
(22) Date of filing: 12.12.1990
(51) Int. Cl.: A47C 27/00, A47C 27/14

(54) **Magnetic cushioning sheet**
Magnetisches Kissen
Coussin magnétique

(30) Priority: 18.01.1990 JP 3760/90 U
(43) Date of publication of application: 31.07.1991
(73) Proprietor: NIHONKENKOZOSHINKENKYUKAI CO. LTD., Fukuoka-shi Fukuoka 813 (JP)
(72) Inventor: Masuda, Isamu, Fukuoka-shi, Fukuoka 814-01 (JP)
(74) Representative: Dipl.-Phys.Dr. Manitz Dipl.-Ing. Finsterwald Dipl.-Ing. Grämkow Dipl.Chem.Dr. Heyn Dipl.Phys. Rotermund Morgan, B.Sc.(Phys.)

(56) References cited:
- EP-A- 0 144 452
- EP-A- 0 263 065
- JP-A- 5 969 004

## Description

### TECHNICAL FIELD

The invention relates to a magnetic cushioning sheet composed of cushioning material having an undulating surface comprising protrusions and indentations therebetween, there being permanent magnets disposed in at least some of these indentations.

### TECHNICAL BACKGROUND

A magnetic cushioning sheet of the above-named kind is known from EP-A-0 144 452 in which the cushioning sheet has the structure of a cushioning material, such as flexible polyurethane foam, comprising a plurality of permanent magnets distributed in alignment on its surface. A plurality of plugs or protrusions are defined by the undulating surface of this material and each permanent magnet is fixed with adhesive at the adjoining concave portion or indentation. The cushioning sheet is piled on a certain core material and its entire contour is covered with cloth to form a mattress or a pillow.

A non-magnetic cushioning sheet is known from JP-A-5 969 004 composed of cushioning material having an undulating surface comprising protrusions and indentations therebetween, in which the cushioning material in the region of the protrusions is harder than the underlying cushioning material as a result of impregnation of this region with polyurethane resin.

The plugs in the magnetic cushioning sheet of the initially named kind will form vacancies or gaps between the user's body and the pillow or mattress by sustaining the body with their plugs providing space for ventilation and dehumidification. At the same time, this aims to give some finger-pressing massaging effect.

However, these plugs tend to be squashed under the weight of the body, and the finger-pressing massaging effect is hardly to be expected. Also the space for ventilation and consequently for dehumidification is difficult to obtain. An idea was therefore offered as a remedy, namely of impregnating rubber latex to the whole cushioning material and applying a hardening process later. Such a cushioning material, if used in a mattress, would not be squashed under the weight of the body thereby ensuring sufficient space for ventilation and dehumidification. A finger-pressing effect is also to be expected.

The prior art kind of cushioning material, if used as a mattress, decreases the cushioning effect to a large extent and obstructs sound sleeping. Moreover, there is a large vacant space between the user's body and the permanent magnets in the concave portion of the sheet, because the plugs do not follow the movement of the user's body with the result that the magnetic lines of force do not effectively act on the body. It is therefore necessary to make the plug height low or to use larger size permanent magnets. However, such a remedy leaves insufficient space for ventilation and weaken the finger-pressing massaging effect. Large size permanent magnets will result in making the cushioning material too heavy.

The object of the present invention is to remedy the problems discussed above and to assure appropriate cushioning effect, to make plugs or protrusions which can follow the movement of the body and to offer a cushioning sheet having a magnetic line of force which acts effectively on the user's body.

### BRIEF DESCRIPTION OF THE INVENTION

The object is satisfied in a magnetic cushioning sheet of the above named kind in that in the region of the protrusions at least a substantial part of the cushioning material is harder than the underlying cushioning material so that each one of the protrusions is barely deformed under the weight of the user's body and sinks into the underlying cushioning material resulting in a reduction of the distance between the user's body and the permanent magnets giving an effective magnetic line of force to the user, and in that the cushioning material is impregnated with latex. In a preferred embodiment of the invention the cushioning material in the region of the protrusions is impregnated with a higher density of latex than the underlying cushioning material.

According to the invention, the entire cushioning material is processed to a semi-hardened state while the plugs are processed later to a higher degree of hardness. If a body applies weight onto this cushioning material, it will be compressed to be deformed in sustaining the load cushioningly. And all plugs will follow the movement of the user's body to give a comfortable feel for sleeping as a result of the cushioning effect of the sheet. Moreover, plugs will not be deformed under the weight, being depressed appropriately into the cushioning sheet to narrow the distances between the permanent magnets and the user's body, thus giving an effective magnetic line of force to the body. Therefore, it is not required to lower the height of the plugs nor to make the size of the permanent magnets any larger.

This assures a good ventilation and finger-pressing massaging effect, and no inconvenience is accompanied by the cushioning material being too heavy.

### BRIEF DESCRIPTION OF THE DRAWINGS

Fig. 1 is a perspective view of the mattress using the magnetic cushioning sheet according to the present invention with its portion broken away.

Fig. 2 is a cross-sectional view taken along the line II - II in Fig. 1.

Fig. 3 is a perspective view of a pillow using the magnetic cushioning sheet according to the present invention with its portion broken away.

Fig. 4 is a cross-sectional view taken along the line IV - IV in Fig. 3.

Fig. 5 is an enlarged sectional view of the magnetic cushioning sheet.

Fig. 6 is a schematic view showing the manufacturing method of the magnetic cushioning sheet.

### PREFERRED EMBODIMENT OF THE INVENTION

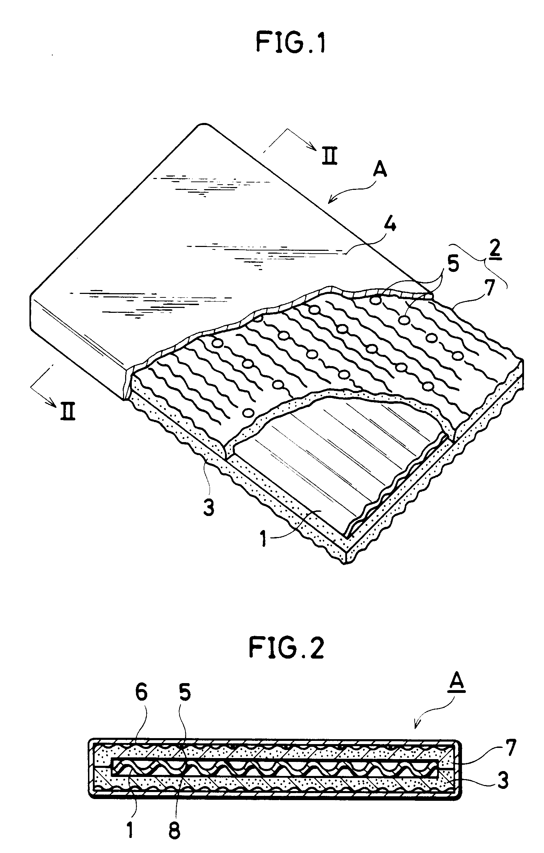

Fig. 1 and 2 show the mattress A for health promotion wherein a corrugated sheet 1 made of synthetic resin such as polyvinyl chloride is placed as the middle layer, upon which a magnetic cushioning sheet 2 and under which other cushioning sheets 3 are disposed respectively, and these cushioning sheets 2 and 3 are enclosed in a covering cloth 4, with their peripheral vertical brims being pasted.

The above-mentioned cushioning sheet 2 is composed of a multiple number of plastic permanent magnets 5 and a cushioning sheet 7 having a multiple number of plugs 6 on its continuous undulating surface. These permanent magnets 5 are fixed with adhesives on the sheet surface of the cushioning material 7 at the concave portion 8 between adjoining plugs.

The above-mentioned cushioning material 7 made of flexible polyurethane foam of continuous pores is of half-way hardened quality having been hardened by impregnating with rubber latex to its entirety and the plugs 6 are processed to hardening later to a higher degree by impregnating rubber latex once again.

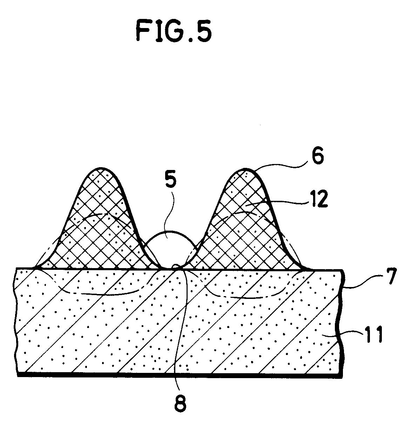

Fig. 5 shows the structure of the cushioning material 7 magnified, wherein numeral 11 indicates the first portion of polyurethane foam having been subjected to a first hardening process and numeral 12 shows the second portion comprising the plugs 6 having been also subjected to a further hardening process being impregnated with rubber latex again. By conducting the hardening processing two times, the second hardened portion 12 is harder than the first hardened portion 11.

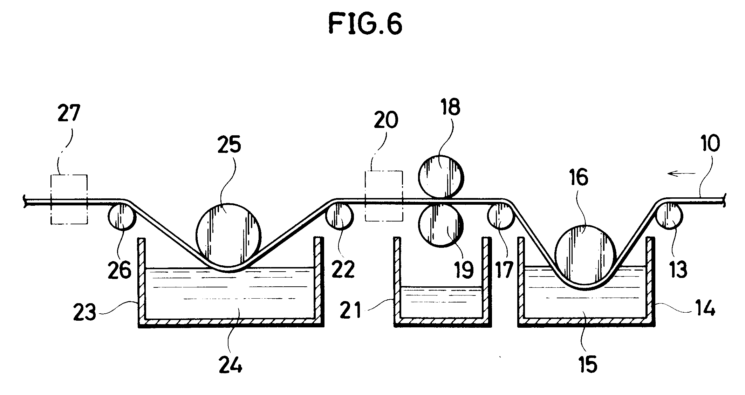

The above-mentioned magnetic cushioning sheet 2 is manufactured by the method shown in Fig. 6.

In this figure, numeral 10 indicates a band-form sheet material of flexible polyurethane foam of continuous pores. This sheet material 10 is led to the first liquid tank 14 after being extruded via the guide roller 13. The liquid tank 14 is filled with rubber latex 15, which impregnates the sheet material 10 by passing under the impregnating roller 16. After this impregnating processing, the sheet material 10 is led to the squeezing rollers 18 and 19 via the guide roller 17. By passing through the squeezing rollers 18 and 19, the quantity of impregnation of rubber latex is controlled. By passing through the heating furnace 20, after the control of the quantity of impregnation of the rubber latex is made, the rubber latex is hardened to a semi-rigid condition, which forms the above-mentioned first hardened portion 11. Numeral 21 in Fig. 6 is the tank for collecting the sqeezed rubber latex.

The sheet material 10 having finished this hardening process is led through the guide roller 22 into the second liquid tank 23. In the liquid tank 23, the rubber latex 24 is always filled to a certain constant level. When the sheet 10 passes under the impregnating roller 25, only the plugs and more particularly the entire plug bodies get impregnated with rubber latex. After this impregnating process, the sheet 10 is led to the heating furnace 27 via the guide roller 26. The rubber latex impregnated in each plug is hardened by heating further to a higher degree forming the second hardened portion 12.

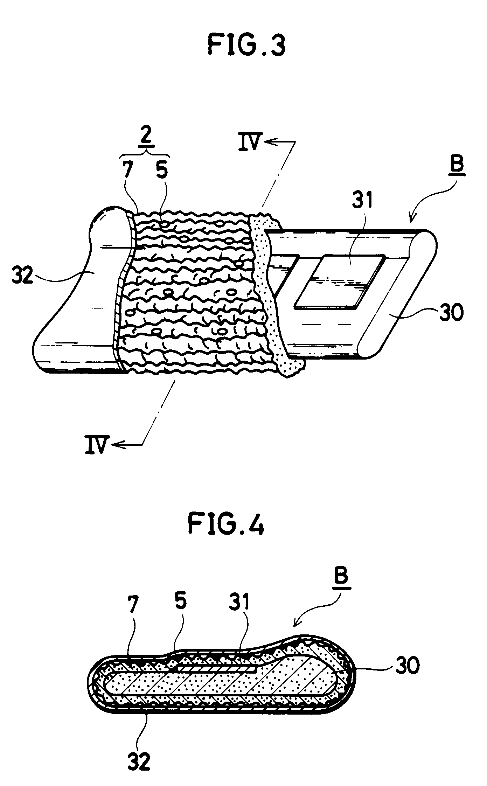

Figs. 3 and 4 show the pillow B for health promoting purpose, which contains a core cushioning material 30 and tile-shaped porcelain plate 31 covered with the magnetic cushioning sheet 2 and its entire body is covered with cloth 32.

The above-mentioned magnetic cushioning sheet 2 has the same composition as the afore-said mattress A which contains the same magnetic cushioning sheet 2, being composed of the cushioning material 7 and a multiple number of permanent magnets 5.

### UTILITIES FOR APPLICATION TO INDUSTRIES

When a user lies on the mattress A of abovementioned construction, the cushioning material 7 being entirely formed in semi-rigid quality, it gives an appropriate cushioning effect to the body as the cushioning material 7 is depressed and deformed to suit the contours of the body, and its plugs 6 press each part of the body in a finger-pressing massaging manner with their higher rigidity. Even when the body moves on the mattress A, all plugs 6 will follow fittingly as the body moves, giving a comfortable feeling for sleeping. Each one of the plugs themselves will be barely deformed under the weight of body and sinks properly into the cushioning base material, which will result in narrowing the distances between the body and the permanent magnets 5 which are positioned in the indented portion of the cushioning material 7, giving an effective magnetic line of force to the user. It is needless to say that the same effects are given as mentioned in the above when the head is rested on the pillow B.

## Claims

1. A magnetic cushioning sheet composed of cushioning material (7) having an undulating surface comprising protrusions (6) and indentations therebetween, there being permanent magnets (5) disposed in at least some of these indentations,
characterized in that in the region of the protrusions (6) at least a substantial part of the cushioning material is harder than the underlying cushioning material so that each one of the protrusions (6) is barely deformed under the weight of the user's body and sinks into the underlying cushioning material resulting in a reduction of the distance between the user's body and the permanent magnets (5) giving an effective magnetic line of force to the user and in that the cushioning material is impregnated with latex.

2. A magnetic cushioning sheet in accordance with claim 1,
characterized in that
in the region of the protrusions (6) the cushioning material is impregnated with a higher density of latex than the underlying cushioning material.

## Patentansprüche

1. Eine magnetische Polsterlage, die aus Polstermaterial (7) mit einer welligen Oberfläche besteht, die Vorsprünge (6) und Vertiefungen dazwischen umfaßt, wobei Permanentmagnete (5) in wenigstens einigen dieser Vertiefungen angeordnet sind,
**dadurch gekennzeichnet,**
daß in dem Bereich der Vorsprünge (6) wenigstens ein wesentlicher Teil des Polstermaterials härter als das darunterliegende Polstermaterial ist, so daß jeder der Vorsprünge (6) kaum unter dem Gewicht des Körpers des Benutzers verformt wird und in das darunterliegende Polstermaterial sinkt, wodurch eine Reduzierung des Abstands zwischen dem Körper des Benutzers und den Permanentmagneten (5) resultiert, was dem Benutzer eine wirksame magnetische Kraftlinie bietet, und daß das Polstermaterial mit Latex imprägniert ist.

2. Eine magnetische Polsterlage nach Anspruch 1,
dadurch gekennzeichnet,
daß in dem Bereich der Vorsprünge (6) das Polstermaterial mit einer höheren Latexdichte imprägniert ist als das darunterliegende Polstermaterial.

## Revendications

1. Feuille amortisseuse magnétique composée d'un matériau amortisseur (7) qui présente une surface ondulée comprenant des saillies (6) et des creux entre ces saillies, des aimants permanents (5) étant disposés dans au moins certains de ces creux, caractérisée en ce que dans la région des saillies (6) au moins une partie substantielle du matériau amortisseur est plus dure que le matériau amortisseur sous-jacent, de sorte que chacune des saillies (6) est simplement déformée sous le poids du corps de l'utilisateur et s'enfonce dans le matériau amortisseur sous-jacent, dont résulte une réduction de la distance entre le corps de l'utilisateur et les aimants permanents (5), en exerçant des lignes de force magnétiques effectives à l'utilisateur, et en ce que le matériau amortisseur est imprégné avec du latex.

2. Feuille amortisseuse magnétique selon la revendication 1, caractérisée en ce que dans la région des saillies (6) le matériau amortisseur est imprégné avec une densité de latex supérieure au matériau amortisseur sous-jacent.
